# EUROPEAN PATENT SPECIFICATION

(11) **EP 3 167 293 B1**
(45) Date of publication and mention of the grant of the patent: **18.03.2020**
(21) Application number: 15819454.8
(22) Date of filing: 13.07.2015
(51) Int. Cl.: G01N 33/68

(54) **SRM/MRM ASSAY FOR THE GTPASE KRAS PROTEIN (KRAS)**
SRM-/MRM-TEST FÜR DAS GTPASE-KRAS-PROTEIN (KRAS)
DOSAGE PAR SRM/MRM DE LA PROTÉINE KRAS GTPASE (KRAS)

(30) Priority: 11.07.2014 US 201462023683 P
(43) Date of publication of application: 17.05.2017
(73) Proprietor: Expression Pathology, Inc., Rockville, MD 20850 (US)
(72) Inventor: KRIZMAN, David, B., Gaithersburg, MD 20882 (US); HEMBROUGH, Todd, Gaithersburg, MD 20882 (US); THYPARAMBIL, Sheeno, Frederick, MD 21704 (US); LIAO, Wei-Li, Herndon, VA 20170 (US)
(74) Representative: AWA Sweden AB
(86) International application number: PCT/US2015/040208
(87) International publication number: WO 2016/007963

(56) References cited:
- WO-A2-2012/099881
- US-A1- 2005 153 380
- US-A1- 2013 068 943
- US-A1- 2013 289 142
- US-A1- 2014 011 748
- US-A1- 2014 011 748
- PATRICK J. HALVEY ET AL: "GeLC-MRM Quantitation of Mutant KRAS Oncoprotein in Complex Biological Samples", JOURNAL OF PROTEOME RESEARCH., vol. 11, no. 7, 6 July 2012 (2012-07-06), pages 3908-3913, XP055427803, US ISSN: 1535-3893, DOI: 10.1021/pr300161j
- DANIEL V. T. CATENACCI ET AL: "Absolute Quantitation of Met Using Mass Spectrometry for Clinical Application: Assay Precision, Stability, and Correlation with MET Gene Amplification in FFPE Tumor Tissue", PLOS ONE, vol. 9, no. 7, 1 July 2014 (2014-07-01), page e100586, XP055163884, DOI: 10.1371/journal.pone.0100586
- CATENACCI ET AL.: 'Absolute Quantitation of Met Using Mass Spectrometry for Clinical Application: Assay Precision, Stability, and Correlation with MET Gene Amplification in FFPE Tumor Tissue' PLOS ONE vol. 9, no. ISS. 7, 01 July 2014, pages 1 - 14, XP055163884

## Description

Specific peptides derived from subsequences of the GTPase KRas Protein, (also referred to as K-Ras-2, Ki-Ras, and c-K-Ras, and referred to herein as "KRas,") are provided. Various methods for the detection of KRas levels in biological samples are known and disclosed, for example, in patent documents WO 2012/099881 and US 2014/011748 as well as in the publications of Halvey et al. (Halvey, P.J. et al. (2012) J. Proteome Res. 11: 3908-3913) and Catenacci et al. (Catenacci, D.V.T. et al. (2014) PLOS One 9: e100586). The peptide sequence and fragmentation/transition ions for each peptide provided herein are particularly useful in a mass spectrometry-based Selected Reaction Monitoring (SRM) assay, which can also be referred to as a Multiple Reaction Monitoring (MRM) assay, and referred to herein as SRM/MRM. The use of peptides for SRM/MRM quantitative analysis of the KRas protein is described.

This SRM/MRM assay as specified in claim 1 can be used to measure *relative* or *absolute* quantitative levels of the peptide of SEQ ID NO:1 from the KRas protein and therefore provides a method of measuring the amount of the KRas protein in a given protein preparation obtained from a biological sample of formalin-fixed tissue by mass spectrometry.

More specifically, the SRM/MRM assay can measure these peptides directly in complex protein lysate samples prepared from cells procured from cancer patient tissue samples of formalin-fixed tissue. Methods of preparing protein samples from formalin-fixed tissue are described in U.S. Patent No. 7,473,532. The methods described in U.S. Patent No. 7,473,532 may conveniently be carried out using Liquid Tissue reagents and protocol available from Expression Pathology Inc. (Rockville, MD).

The most widely and advantageously available form of tissues from cancer patients' tissue is formalin-fixed, paraffin-embedded tissue. Formaldehyde/formalin-fixation of surgically removed tissue is by far the most common method of preserving cancer tissue samples worldwide and is the accepted convention for standard pathology practice. Aqueous solutions of formaldehyde are referred to as formalin. "100%" formalin consists of a saturated solution of formaldehyde (about 40% by volume or 37% by mass) in water, with a small amount of stabilizer, usually methanol to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus, molecular analytical methods to analyze formalin fixed cancer tissue will be the most accepted and heavily utilized methods for analysis of cancer patient tissue.

Results from the SRM/MRM assay as specified in claim 1 can be used to correlate accurate and precise quantitative levels of the KRas protein within the specific tissue samples (e.g., cancer tissue sample) of the patient or subject from whom the tissue (biological sample) was collected and preserved. This not only provides diagnostic and prognostic information about the cancer, but also permits a physician or other medical professional to more accurately determine appropriate therapy for the patient. Such an assay that provides diagnostically, prognostically, and therapeutically important information about levels of protein expression in a diseased tissue or other patient sample is termed a *companion diagnostic* assay. For example, such an assay can be designed to diagnose the stage or degree of a cancer and determine a therapeutic agent to which a patient is most likely to respond.

### Summary

The method as specified in claim 1 measures *relative* or *absolute* levels of the KRas fragment peptide of SEQ ID NO:1. The peptide may be modified or unmodified. Examples of modifications include phosphorylated amino acid residues and glycosylated amino acid residues that may be present on the peptides.

*Relative* quantitative levels of the KRas protein can be determined by the SRM/MRM methodology by, for example, comparing SRM/MRM signature peak areas (e.g., signature peak area or integrated fragment ion intensity) of the KRas fragment peptide of SEQ ID NO:1 in different samples. Alternatively, it may also be possible to further compare multiple SRM/MRM signature peak areas for multiple other KRas signature peptides, where each peptide has its own specific SRM/MRM signature peak, to determine the relative KRas protein content in one biological sample and compare it with the KRas protein content in one or more additional or different biological samples. In this way, the amount of a particular peptide, or peptides, from the KRas protein, and therefore the amount of the KRas protein, is determined relative to the same KRas peptide, or peptides, across 2 or more biological samples under the same experimental conditions. In addition, relative quantitation can also be determined for the KRas fragment peptide of SEQ ID NO:1, and for further peptides, if applicable, from the KRas protein within a single sample by comparing the signature peak area for that peptide by SRM/MRM methodology to the signature peak area for another and different peptide, or peptides, from a different protein, or proteins, within the same protein preparation from the biological sample. In this way, the amount of a particular peptide from the KRas protein, and therefore the amount of the KRas protein, is determined relative one to another within the same sample. These approaches permit quantitation of an individual peptide, or peptides, from the KRas protein to the amount of another peptide, or peptides, between samples and within samples wherein the amounts as determined by signature peak area are relative one to another, regardless of the absolute weight to volume or weight to weight amounts of the KRas peptide in the protein preparation from the biological sample. Relative quantitative data about individual signature peak areas between different samples can be normalized to the amount of protein analyzed per sample. Relative quantitation can be performed across many peptides from multiple proteins and the KRas protein simultaneously in a single sample and/or across many samples to gain insight into relative protein amounts of one peptide/protein with respect to other peptides/proteins.

*Absolute* quantitative levels of the KRas protein can be determined by, for example, the SRM/MRM methodology whereby the SRM/MRM signature peak area of the KRas fragment peptide of SEQ ID NO:1 in one biological sample is compared to the SRM/MRM signature peak area of a spiked internal standard. In one embodiment, the internal standard is a synthetic version of the same exact KRas peptide that contains one or more amino acid residues labeled with one or more heavy isotopes. Such an isotope labeled internal standard can be synthesized so that, when analyzed by mass spectrometry, it generates a predictable and consistent SRM/MRM signature peak that is different and distinct from the native KRas peptide signature peak and therefore can be used as a comparator peak. Thus, when the internal standard is spiked into a protein preparation from a biological sample in known amounts and analyzed by mass spectrometry, the SRM/MRM signature peak area of the native peptide is compared to the SRM/MRM signature peak area of the internal standard peptide, and this numerical comparison indicates either the absolute molarity and/or absolute weight of the native peptide present in the original protein preparation from the biological sample. Absolute quantitative data for fragment peptides are displayed according to the amount of protein analyzed per sample. Absolute quantitation can be performed across many peptides, and thus proteins, simultaneously in a single sample and/or across many samples to gain insight into absolute protein amounts in individual biological samples and in entire cohorts of individual samples.

The SRM/MRM assay method described herein can be used to aid diagnosis of the stage of cancer and/or the patient prognosis, for example, directly in patient-derived, formalin-fixed tissue, and to aid in determining which therapeutic agent would be most advantageous for use in treating that patient. Cancer tissue that is removed from a patient either through surgery, such as for therapeutic removal of partial or entire tumors, or through biopsy procedures conducted to determine the presence or absence of suspected disease, is analyzed to determine whether or not a specific protein, or proteins, and which forms of proteins, are present in that patient tissue. Moreover, the expression level of a protein, or multiple proteins, can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues not affected by the cancer.

Assays of protein levels (e.g., KRas levels) can also be used to diagnose the stage of cancer and provide prognostic information about a patient or subject diagnosed with cancer by employing the KRas levels. The level of an individual KRas peptide is defined as the molar amount of the peptide determined by the SRM/MRM assay per total amount of protein lysate analyzed. Information regarding KRas can thus be used to aid in determining stage or grade of a cancer and/or patient prognosis by correlating the level of the KRas protein (or fragment peptides of the KRas protein) with levels observed in normal tissues. Once the stage and/or grade, and/or KRas protein expression characteristics of the cancer has been determined, that information can be matched to a list of therapeutic agents (chemical and biological) developed to specifically treat cancer tissue that is characterized by, for example, abnormal expression of the protein or protein(s) (e.g., KRas) that were assayed. Matching information from a KRas protein assay to a list of therapeutic agents that specifically targets, for example, the KRas protein or cells/tissue expressing the protein, defines what has been termed a *personalized medicine* approach to treating disease. The assay methods described herein form the foundation of a *personalized medicine* approach by using analysis of proteins from the patient's own tissue as a source for diagnostic and treatment decisions.

### Brief Description of the Drawings

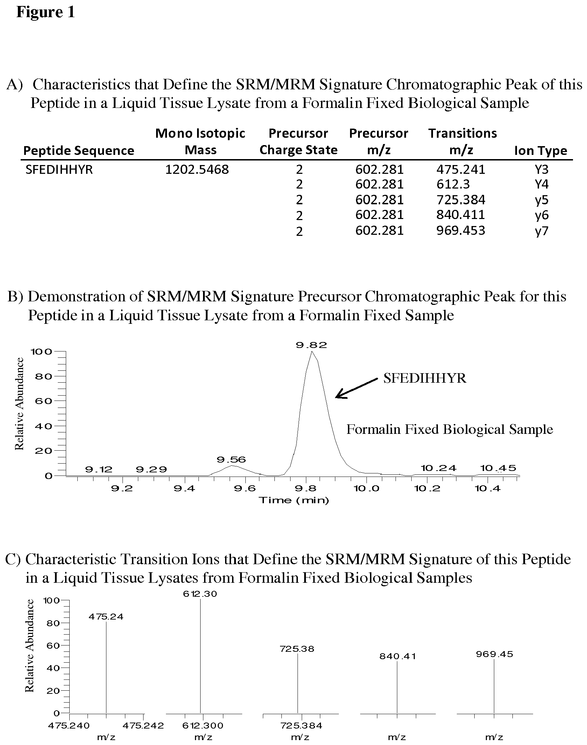

Figure 1, parts A to C, shows an example of an SRM/MRM assay of the KRas fragment peptide of SEQ ID NO: 1 performed on a Liquid Tissue lysate from a formalin fixed biological sample with quantitation of the KRas peptide conducted on a triple quadrupole mass spectrometer. The specific characteristics about how to measure this peptide in biological samples that have been fixed in formalin are shown.

### Detailed Description

The KRas fragment peptide of SEQ ID NO:1 is used as a surrogate reporter to determine the abundance of KRas protein in a sample using a mass spectrometry-based SRM/MRM assay.

KRas fragment peptides may be generated by a variety of methods including by the use of the Liquid Tissue protocol provided in US Patent 7,473,532. The Liquid Tissue protocol and reagents are capable of producing peptide samples suitable for mass spectroscopic analysis from formalin-fixed, paraffin-embedded tissue by proteolytic digestion of the proteins in the tissue/biological sample. In the Liquid Tissue protocol the tissue/biological is heated in a buffer for an extended period of time (e.g., from about 80° C to about 100° C for a period of time from about 10 minutes to about 4 hours) to reverse or release protein cross-linking. The buffer employed is a neutral buffer, (e.g., a Tris-based buffer, or a buffer containing a detergent). Following heat treatment, the tissue/biological sample is treated with one or more proteases, including trypsin, chymotrypsin, pepsin, and endoproteinase Lys-C for a time sufficient to disrupt the tissue and cellular structure of the biological sample and to liquefy the sample (e.g., a period of time from 30 minutes to 24 hours at a temperature from 37° C to 65° C). The result of the heating and proteolysis is a liquid, soluble, dilutable biomolecule lysate.

Surprisingly, it has been found that many potential peptide sequences from the KRas protein are unsuitable or ineffective for use in mass spectrometry-based SRM/MRM assays for reasons that are not immediately evident. This is particularly true for peptides derived from formalin fixed tissue. As it was not possible to predict the most suitable peptides for MRM/SRM assay, it was necessary to experimentally identify suitable peptides in actual Liquid Tissue lysates to develop a reliable and accurate SRM/MRM assay for the KRas protein. While not wishing to be bound by any theory, it is believed that some peptides might, for example, be difficult to detect by mass spectrometry as they do not ionize well or produce fragments that are not distinct from other proteins. Peptides may also fail to resolve well in separation (e.g., liquid chromatography), or may adhere to glass or plastic ware.

The KRas fragment peptide of SEQ ID NO:1 and further KRas peptides identified (e.g., Tables 1 and 2) were derived from the KRas protein by protease digestion of all the proteins within a complex Liquid Tissue lysate prepared from cells procured from formalin-fixed cancer tissue. Unless noted otherwise, in each instance the protease was trypsin. The Liquid Tissue lysate was then analyzed by mass spectrometry to determine those peptides derived from the KRas protein that are detected and analyzed by mass spectrometry. Identification of a specific preferred subset of peptides for mass-spectrometric analysis is based on; 1) experimental determination of which peptide or peptides from a protein ionize in mass spectrometry analyses of Liquid Tissue lysates, and 2) the ability of the peptide to survive the protocol and experimental conditions used in preparing a Liquid Tissue lysate. This latter property extends not only to the amino acid sequence of the peptide but also to the ability of a modified amino acid residue within a peptide to survive in modified form during the sample preparation.

Protein lysates from cells procured directly from formalin (formaldehyde) fixed tissue were prepared using the Liquid Tissue reagents and protocol that entails collecting cells into a sample tube via tissue microdissection followed by heating the cells in the Liquid Tissue buffer for an extended period of time. Once the formalin-induced cross linking has been negatively affected, the tissue/cells are then digested to completion in a predictable manner using a protease, such as, for example, trypsin, although other proteases can be used. Each protein lysate is turned into a collection of peptides by digestion of intact polypeptides with the protease. Each Liquid Tissue lysate was analyzed (e.g., by ion trap mass spectrometry) to perform multiple global proteomic surveys of the peptides where the data was presented as identification of as many peptides as could be identified by mass spectrometry from all cellular proteins present in each protein lysate.

An ion trap mass spectrometer or another form of a mass spectrometer that is capable of performing global profiling for identification of as many peptides as possible from a single complex protein/peptide lysate is employed. Ion trap mass spectrometers however may advantageously be used conducting global profiling of peptides. Although an SRM/MRM assay can be developed and performed on any type of mass spectrometer, including a MALDI, ion trap, or triple quadrupole, advantageously a triple quadrupole instrument platform is used for an SRM/MRM assay. That type of a mass spectrometer is suitable instrument for analyzing a single isolated target peptide within a very complex protein lysate that may consist of hundreds of thousands to millions of individual peptides from all the proteins contained within a cell.

Once as many peptides as possible were identified in a single MS analysis of a single lysate under the conditions employed, then that list of peptides was collated and used to determine the proteins that were detected in that lysate. That process was repeated for multiple Liquid Tissue lysates, and the very large list of peptides was collated into a single dataset. That type of dataset can be considered to represent the peptides that can be detected in the type of biological sample that was analyzed (after protease digestion), and specifically in a Liquid Tissue lysate of the biological sample, and thus includes the peptides for specific proteins, such as for example the KRas protein.

The KRas tryptic peptides identified as useful in the determination of absolute or relative amounts of the KRas protein was the peptide of SEQ ID NO:1. Further suitable peptides are listed in Table 1. Each of those peptides was detected by mass spectrometry in Liquid Tissue lysates prepared from formalin-fixed, paraffin-embedded tissue.

**Table 1**

| **SEQ ID** | **Peptide sequence** |
|---|---|
| SEQ ID NO: 1 | SFEDIHHYR |
| SEQ ID NO: 2 | LVVVGAGGVGK |
| SEQ ID NO: 3 | SALTIQLIQNHFVDEYDPTIEDSYR |
| SEQ ID NO: 4 | QAQDLAR |
| SEQ ID NO: 5 | SYGIPFIETSAK |
| SEQ ID NO: 6 | VEDAFYTLVR |
| SEQ ID NO: 7 | QGVDDAFYTLVR |

The KRas tryptic peptides listed in Table 1 include those detected from multiple Liquid Tissue lysates of multiple different formalin-fixed tissues of different human organs including prostate, colon, and breast. Each of those peptides other than that of SEQ ID NO:1 is also considered useful for quantitative SRM/MRM assay of the KRas protein in formalin fixed tissue. Further data analysis of these experiments indicated no preference for any specific peptides from any specific organ site. Thus, these peptides other than that of SEQ ID NO:1 may also be used for conducting SRM/MRM assays of the KRas protein on a Liquid Tissue lysate from any formalin-fixed tissue originating from any biological sample or from any organ site in the body, but which is not within the scope of the present invention.

In order to most efficiently implement an SRM/MRM assay for the KRas peptide of SEQ ID NO:1 it is desirable to utilize information in addition to the peptide sequence in the analysis. That additional information may be used in directing and instructing the mass spectrometer (e.g. a triple quadrupole mass spectrometer), to perform the correct and focused analysis of specific targeted peptide(s), such that the assay may be effectively performed.

The additional information about target peptides in general, and about specific KRas peptides, may include one or more of the mono isotopic mass of the peptide, its precursor charge state, the precursor m/z value, the m/z transition ions, and the ion type of each transition ion. Table 2 shows additional peptide information that may be used to develop an SRM/MRM assay for the KRas protein for one (1) of the KRas peptides from the list in Table 1. Similar additional information described for the one (1) KRas peptide shown by example in Table 2 may be prepared, obtained, and applied to the analysis of the other peptides contained in Table 1.

**Table 2**

| **SEQ ID** | **Peptide sequence** | **Mono Isotopic Mass** | **Precursor Charge State** | **Precursor m/z** | **Transition m/z** | **Ion Type** |
|---|---|---|---|---|---|---|
| SEQ ID NO: 1 | SFEDIHHYR | 1202.5468 | 2 | 602.281 | 475.241 | y3 |
| | | | 2 | 602.281 | 612.3 | y4 |
| | | | 2 | 602.281 | 725.384 | y5 |
| | | | 2 | 602.281 | 840.411 | y6 |
| | | | 2 | 602.281 | 969.453 | y7 |

The method described below was used to: 1) identify candidate peptides from the KRas protein that can be used for a mass spectrometry-based SRM/MRM assay for the KRas protein, 2) develop an individual SRM/MRM assay, or assays, for target peptides from the KRas protein in order to correlate and 3) apply quantitative assays to cancer diagnosis and/or choice of optimal therapy.

### Assay Method

1. Identification of SRM/MRM candidate fragment peptides for the KRas protein
   a. Prepare a Liquid Tissue protein lysate from a formalin fixed biological sample using a protease or proteases, (that may or may not include trypsin), to digest proteins
   b. Analyze all protein fragments in the Liquid Tissue lysate on an ion trap tandem mass spectrometer and identify all fragment peptides from the KRas protein, where individual fragment peptides do not contain any peptide modifications such as phosphorylations or glycosylations
   c. Analyze all protein fragments in the Liquid Tissue lysate on an ion trap tandem mass spectrometer and identify all fragment peptides from the KRas protein that carry peptide modifications such as for example phosphorylated or glycosylated residues
   d. All peptides generated by a specific digestion method from the entire, full length KRas protein potentially can be measured, but preferred peptides used for development of the SRM/MRM assay are those that are identified by mass spectrometry directly in a complex Liquid Tissue protein lysate prepared from a formalin fixed biological sample
   e. Peptides that are specifically modified (phosphorylated, glycosylated, etc.) in patient tissue and which ionize, and thus detected, in a mass spectrometer when analyzing a Liquid Tissue lysate from a formalin fixed biological sample are identified as candidate peptides for assaying peptide modifications of the KRas protein
2. Mass Spectrometry Assay for Fragment Peptides from KRas Protein
   a. SRM/MRM assay on a triple quadrupole mass spectrometer for individual fragment peptides identified in a Liquid Tissue lysate is applied to peptides from the KRas protein
      i. Determine optimal retention time for a fragment peptide for optimal chromatography conditions including liquid chromatography, nano-reversed phase liquid chromatography, high performance liquid chromatography, and reverse phase high performance liquid chromatography
      ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
      iii. SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer
   b. Perform SRM/MRM analysis so that the amount of the fragment peptide of the KRas protein that is detected, as a function of the unique SRM/MRM signature peak area from an SRM/MRM mass spectrometry analysis, can indicate both the relative and absolute amount of the protein in a particular protein lysate.
      i. Relative quantitation may be achieved by:
         1. Determining increased or decreased presence of the KRas protein by comparing the SRM/MRM signature peak area from a given KRas peptide detected in a Liquid Tissue lysate from one formalin fixed biological sample to the same SRM/MRM signature peak area of the same KRas fragment peptide in at least a second, third, fourth or more Liquid Tissue lysates from least a second, third, fourth or more formalin fixed biological samples
         2. Determining increased or decreased presence of the KRas protein by comparing the SRM/MRM signature peak area from a given KRas peptide detected in a Liquid Tissue lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
         3. Determining increased or decreased presence of the KRas protein by comparing the SRM/MRM signature peak area for a given KRas peptide to the SRM/MRM signature peak areas from other fragment peptides derived from different proteins within the same Liquid Tissue lysate from the formalin fixed biological sample in order to normalize changing levels of KRas protein to levels of other proteins that do not change their levels of expression under various cellular conditions.
         4. These assays can be applied to both unmodified fragment peptides and for modified fragment peptides of the KRas protein, where the modifications include but are not limited to phosphorylation and/or glycosylation, and where the relative levels of modified peptides are determined in the same manner as determining relative amounts of unmodified peptides.
      ii. Absolute quantitation of a given peptide may be achieved by comparing the SRM/MRM signature peak area for a given fragment peptide from the KRas protein in an individual biological sample to the SRM/MRM signature peak area of an internal fragment peptide standard spiked into the protein lysate from the biological sample
         1. The internal standard is a labeled synthetic version of the fragment peptide from the KRas protein that is being interrogated. This standard is spiked into a sample in known amounts, and the SRM/MRM signature peak area can be determined for both the internal fragment peptide standard and the native fragment peptide in the biological sample separately, followed by comparison of both peak areas
         2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.
3. Apply Fragment Peptide Quantitation to Cancer Diagnosis and Treatment
   a. Perform relative and/or absolute quantitation of fragment peptide levels of the KRas protein and demonstrate that the previously-determined association, as well understood in the field of cancer, of KRas protein expression to the stage/grade/status of cancer in patient tumor tissue is confirmed
   b. Perform relative and/or absolute quantitation of fragment peptide levels of the KRas protein and demonstrate correlation with clinical outcomes from different treatment strategies, wherein this correlation has already been demonstrated in the field or can be demonstrated in the future through correlation studies across cohorts of patients and tissue from those patients. Once either previously established correlations or correlations derived in the future are confirmed by this assay then the assay method can be used to determine optimal treatment strategy

Figure 1 shows an example of a single SRM/MRM assay as specified in claim 1 performed on a Liquid Tissue lysate from a formalin-fixed biological sample. An SRM/MRM assay was developed for the peptide of SEQ ID NO:1 for quantitation of the KRas protein on a triplequadrupole mass spectrometer. Specific and unique characteristics about this KRas peptide of SEQ ID NO:1 (sequence SFEDIHHYR) were developed by analysis of all KRas peptides on both an ion trap and triple quadrupole mass spectrometers and are shown in Figure 1A. That information includes the monoisotopic mass of the peptide, its precursor charge state, the precursor m/z value, the transition m/z values of the precursor, and the ion types of each of the identified transitions. That information must be determined experimentally for each and every candidate SRM/MRM peptide directly in Liquid Tissue lysates from formalin-fixed samples/tissue, because, interestingly, not all peptides from the KRas protein can be detected in such lysates using SRM/MRM as described herein, indicating that KRas peptides not detected cannot be considered candidate peptides for developing an SRM/MRM assay for use in quantitating peptides/proteins directly in Liquid Tissue lysates from formalin fixed samples/tissue.

As shown in Figure 1B, this particular SRM/MRM assay was performed on a triple quadrupole mass spectrometer. The experimental sample in this experiment was a Liquid Tissue protein lysate prepared from a cell line that had been formalin-fixed, paraffin-embedded to act as a tissue surrogate. Data from the assay indicate the presence of the unique SRM/MRM signature peak for the KRas peptide of SEQ ID NO:1 in the formalin-fixed sample.

Figure 1C shows the specific transition ion characteristics for this peptide used to quantitatively measure of the above-mentioned peptide in formalin fixed biological samples. These data indicate absolute amounts of this KRas peptide as a function of the molar amount of the peptide per microgram of protein lysate analyzed. Assessment of KRas protein levels in tissues based on analysis of formalin fixed patient-derived tissue can provide diagnostic, prognostic, and therapeutically-relevant information about each particular patient.

This disclosure describes a method for measuring the level of the GTPase KRas Protein in a biological sample of formalin-fixed tissue, comprising detecting and quantifying the amount of a KRas fragment peptide in a protein digest prepared from the biological sample using mass spectrometry; and calculating the level of KRas protein in the sample; wherein said KRas fragment peptide is the peptide of SEQ ID NO:1; and wherein the level is a relative level or an absolute level. In one embodiment, quantifying the KRas fragment peptide comprises determining the amount of the KRas fragment peptide of SEQ ID NO:1 in a biological sample by comparison to an added internal standard peptide of known amount, wherein the KRas fragment peptide in the biological sample is compared to an internal standard peptide having the same amino acid sequence. In some embodiments, the internal standard is an isotopically labeled internal standard peptide comprises one or more heavy stable isotopes selected from ¹⁸O, ¹⁷O, ³⁴S, ¹⁵N, ¹³C, ²H, and combinations thereof.

The method for measuring the level of the KRas protein in a biological sample described herein may be used as a diagnostic and/or prognostic indicator of cancer in a patient or subject. In one embodiment, the results from measurements of the level of the KRas protein may be employed to determine the diagnostic stage/grade/status and/or the prognostic status of a cancer by correlating (e.g., comparing) the level of KRas protein found in a tissue with the level of that protein found in normal and/or cancerous or precancerous tissues.

Because both nucleic acids and protein can be analyzed from the same Liquid Tissue™ biomolecular preparation it is possible to generate additional information about disease diagnosis and drug treatment decisions from the nucleic acids in same sample upon which proteins were analyzed. For example, if the KRas protein is expressed by certain cells at increased levels, when assayed by SRM the data can provide information about the state of the cells and their potential for uncontrolled growth, potential drug resistance and the development of cancers can be obtained. At the same time, information about the status of the KRas genes and/or the nucleic acids and proteins they encode (e.g., mRNA molecules and their expression levels or splice variations) can be obtained from nucleic acids present in the same Liquid Tissue™ biomolecular preparation can be assessed simultaneously to the SRM analysis of the KRas protein. Any gene and/or nucleic acid not from the KRas and which is present in the same biomolecular preparation can be assessed simultaneously to the SRM analysis of the KRas protein. In one instance, information about the KRas protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including single base pair polymorphisms, transitions, transversions, and combinations thereof.

## Claims

1. A method for measuring the level of the GTPase KRas Protein (KRas) in a human biological sample of formalin-fixed tissue, comprising detecting and quantifying the amount of a KRas fragment peptide in a protein digest prepared from said biological sample using mass spectrometry; and calculating the level of KRas protein in said sample; wherein the KRas fragment peptide is the peptide of SEQ ID NO:1; and wherein said level is a relative level or an absolute level.

2. The method of claim 1, further comprising the step of fractionating said protein digest prior to detecting and quantifying the amount of said KRas fragment peptide, wherein said fractionating step is selected from the group consisting of liquid chromatography, nano-reversed phase liquid chromatography, high performance liquid chromatography, and reverse phase high performance liquid chromatography.

3. The method of claim 1 or 2, wherein said protein digest comprises a protease digest.

4. The method of any of claims 1 to 3, wherein the mode of mass spectrometry used is Selected Reaction Monitoring (SRM), Multiple Reaction Monitoring (MRM), and/or multiple Selected Reaction Monitoring (mSRM).

5. The method of any of claims 1 to 4, wherein the tissue is paraffin embedded tissue.

6. The method of claim 5, wherein the tissue is obtained from a tumor.

7. The method of any of claims 1 to 6, wherein quantifying the KRas fragment peptide comprises comparing the amount of said KRas fragment peptide in one biological sample to the amount of the same KRas fragment peptide in a different and separate biological sample.

8. The method of claim 7, wherein quantifying said KRas fragment peptide comprises determining the amount of said KRas fragment peptide in a biological sample by comparison to an added internal standard peptide of known amount, wherein said KRas fragment peptide in the biological sample is compared to an internal standard peptide having the same amino acid sequence.

9. The method of claim 8, wherein the internal standard peptide is an isotopically labeled peptide comprising one or more heavy stable isotopes selected from ¹⁸O, ¹⁷O, ³⁴S, ¹⁵N, ¹³C, ²H and combinations thereof.

10. The method of any of claims 1 to 9, wherein detecting and quantifying the amount of said KRas fragment peptide in the protein digest indicates the presence of KRas protein and an association with cancer in the subject from whom the biological sample was obtained.

11. The method of claim 10, further comprising correlating the results of said detecting and quantifying the amount of said KRas fragment peptide, or the level of said KRas protein to the diagnostic stage/grade/status of the cancer.

12. The method of claim 11, wherein correlating the results of said detecting and quantifying the amount of said KRas fragment peptide, or the level of said KRas protein to the diagnostic stage/grade/status of the cancer is combined with detecting and/or quantifying the amount of other proteins or peptides from other proteins in a multiplex format to provide additional information about the diagnostic stage/grade/status of the cancer.

13. The method of any one of claims 1 to 12, further comprising selecting for the subject from which said biological sample was obtained a therapeutic agent based on the presence, absence, or amount of said KRas fragment peptide or the level of KRas protein.

14. The method of claim 13, wherein said therapeutic agent binds the KRas protein and/or inhibits its biological activity.

15. The method of claim 14, wherein the therapeutic agent is Reolysin.

## Patentansprüche

1. Verfahren zum Messen der Konzentration des GTPase-KRas-Proteins (KRas) in einer humanen biologischen Probe von formalinfixiertem Gewebe, bei dem man die Menge eines KRas-Fragmentpeptids in einem von der biologischen Probe hergestellten Proteinverdau unter Anwendung von Massenspektrometrie nachweist und quantifiziert und die Konzentration des KRas-Proteins in der Probe berechnet, wobei es sich bei dem KRas-Fragmentpeptid um das Peptid der SEQ ID NO: 1 handelt und wobei es sich bei der Konzentration um eine relative Konzentration oder eine absolute Konzentration handelt.

2. Verfahren nach Anspruch 1, weiterhin umfassend den Schritt der Fraktionierung des Proteinverdaus vor dem Nachweis und der Quantifizierung der Menge des KRas-Fragmentpeptids, wobei der Fraktionierungsschritt aus der aus Flüssigchromatographie, Nanoumkehrphasenflüssigchromatographie, Hochleistungsflüssigchromatographie und Umkehrphasenhochleistungsflüssigchromatographie bestehenden Gruppe ausgewählt ist.

3. Verfahren nach Anspruch 1 oder 2, wobei der Pverdau einen Proteaseverdau umfasst.

4. Verfahren nach einem der Ansprüche 1 bis 3, wobei es sich bei dem angewendeten Massenspektrometriemodus um Selected Reaction Monitoring (SRM), Multiple Reaction Monitoring (MRM) und/oder Multiple Selected Reaction Monitoring (mSRM) handelt.

5. Verfahren nach einem der Ansprüche 1 bis 4, wobei es sich bei dem Gewebe um in Paraffin eingebettetes Gewebe handelt.

6. Verfahren nach Anspruch 5, wobei das Gewebe von einem Tumor erhalten wird.

7. Verfahren nach einem der Ansprüche 1 bis 6, wobei die Quantifizierung des KRas-Fragmentpeptids den Vergleich der Menge des KRas-Fragmentpeptids in einer biologischen Probe mit der Menge des gleichen KRas-Fragmentpeptids in einer verschiedenen und separaten biologischen Probe umfasst.

8. Verfahren nach Anspruch 7, wobei das Quantifizieren des KRas-Fragmentpeptids die Bestimmung der Menge des KRas-Fragmentpeptids in einer biologischen Probe durch Vergleich mit einem zugefügten Interner-Standard-Peptid einer bekannten Menge umfasst, wobei man das KRas-Fragmentpeptid in der biologischen Probe mit einem Interner-Standard-Peptid mit der gleichen Aminosäuresequenz vergleicht.

9. Verfahren nach Anspruch 8, wobei es sich bei dem Interner-Standard-Peptid um ein isotopenmarkiertes Peptid handelt, das einen oder mehrere schwere stabile Isotope ausgewählt aus ¹⁸O, ¹⁷O, ³⁴S, ¹⁵N, ¹³C, ²H und Kombinationen davon umfasst.

10. Verfahren nach einem der Ansprüche 1 bis 9, wobei der Nachweis und die Quantifizierung der Menge des KRas-Fragmentpeptids in dem Proteinverdau das Vorhandensein von KRas-Protein und einer Assoziation mit Krebs in dem Subjekt, von dem die biologische Probe erhalten wurde, anzeigt.

11. Verfahren nach Anspruch 10, weiterhin umfassend das Korrelieren der Ergebnisse des Nachweises und der Quantifizierung der Menge des KRas-Fragmentpeptids oder der Konzentration des KRas-Proteins mit dem diagnostischen Stadium/Grad/Status der Krebserkrankung.

12. Verfahren nach Anspruch 11, wobei man das Korrelieren der Ergebnisse des Nachweises und der Quantifizierung der Menge des KRas-Fragmentpeptids oder der Konzentration des KRas-Proteins mit dem diagnostischen Stadium/Grad/Status der Krebserkrankung mit dem Nachweis und/oder der Quantifizierung der Menge anderer Proteine oder Peptide von anderen Proteinen in einem Multiplexformat kombiniert, unter Bereitstellung zusätzlicher Informationen über das diagnostische Stadium/den diagnostischen Grad/den diagnostischen Status der Krebserkrankung.

13. Verfahren nach einem der Ansprüche 1 bis 12, bei dem man weiterhin für das Subjekt, von dem die biologische Probe erhalten wurde, basierend auf dem Vorhandensein, der Abwesenheit oder der Menge des KRas-Fragmentpeptids oder der Konzentration an KRas-Protein ein Therapeutikum auswählt.

14. Verfahren nach Anspruch 13, wobei das Therapeutikum an das KRas-Protein bindet und/oder dessen biologische Aktivität hemmt.

15. Verfahren nach Anspruch 14, wobei das Therapeutikum Reolysin ist.

## Revendications

1. Procédé pour la mesure du niveau de la protéine GTPase KRas (KRas) dans un échantillon biologique humain de tissu fixé au formol, comprenant la détection et la quantification de la quantité d'un fragment peptidique de KRas dans une digestion de protéines préparée à partir dudit échantillon biologique à l'aide de spectrométrie de masse ; et le calcul du niveau de protéine KRas dans ledit échantillon ; ledit fragment peptidique de KRas étant le peptide de SEQ ID NO : 1 ; et ledit niveau étant un niveau relatif ou un niveau absolu.

2. Procédé selon la revendication 1, comprenant en outre l'étape de fractionnement de ladite digestion de protéines avant la détection et la quantification de la quantité dudit fragment peptidique de KRas, ladite étape de fractionnement étant choisie dans le groupe constitué par la chromatographie liquide, la nano-chromatographie liquide en phase inverse, la chromatographie liquide à haute performance, et la chromatographie liquide à haute performance en phase inverse.

3. Procédé selon la revendication 1 ou 2, ladite digestion de protéines comprenant une digestion de protéases.

4. Procédé selon l'une quelconque des revendications 1 à 3, le mode de spectrométrie de masse utilisé étant le suivi de réaction sélectionnée (SRS), le suivi de réactions multiples (SRM), et/ou le suivi de réactions sélectionnées multiples (SRSm).

5. Procédé selon l'une quelconque des revendications 1 à 4, le tissu étant un tissu enrobé de paraffine.

6. Procédé selon la revendication 5, le tissu étant obtenu à partir d'une tumeur.

7. Procédé selon l'une quelconque des revendications 1 à 6, la quantification du fragment peptidique de KRas comprenant la comparaison de la quantité dudit fragment peptidique de KRas dans un échantillon biologique avec la quantité du même fragment peptidique de KRas dans un échantillon biologique différent et distinct.

8. Procédé selon la revendication 7, la quantification dudit fragment peptidique de KRas comprenant la détermination de la quantité dudit fragment peptidique de KRas dans un échantillon biologique par comparaison avec un peptide standard interne ajouté de quantité connue, ledit fragment peptidique de KRas dans l'échantillon biologique étant comparé à un peptide standard interne possédant la même séquence d'acides aminés.

9. Procédé selon la revendication 8, le peptide standard interne étant un peptide isotopiquement marqué comprenant un ou plusieurs isotopes stables lourds choisis parmi ¹⁸O, ¹⁷O, ³⁴S, ¹⁵N, ¹³C, ²H et des combinaisons correspondantes.

10. Procédé selon l'une quelconque des revendications 1 à 9, la détection et la quantification de la quantité dudit fragment peptidique de KRas dans la digestion de protéines indiquant la présence de protéine KRas et une association avec un cancer chez le sujet duquel l'échantillon biologique a été obtenu.

11. Procédé selon la revendication 10, comprenant en outre la corrélation des résultats de ladite détection et de ladite quantification de la quantité dudit fragment peptidique de KRas, ou du niveau de ladite protéine KRas avec le diagnostic stade/degré/statut du cancer.

12. Procédé selon la revendication 11, la corrélation des résultats de ladite détection et de ladite quantification de la quantité dudit fragment peptidique de KRas, ou du niveau de ladite protéine KRas avec le diagnostic stade/degré/statut du cancer étant combinée avec la détection et/ou la quantification de la quantité d'autres protéines ou de peptides d'autres protéines dans un format multiplex pour fournir des informations supplémentaires sur le diagnostic stade/degré/statut du cancer.

13. Procédé selon l'une quelconque des revendications 1 à 12, comprenant en outre la sélection, pour le sujet duquel ledit échantillon biologique a été obtenu, d'un agent thérapeutique, basé sur la présence, l'absence, ou la quantité dudit fragment peptidique de KRas ou sur le niveau de ladite protéine KRas.

14. Procédé selon la revendication 13, ledit agent thérapeutique se liant à la protéine KRas et/ou inhibant son activité biologique.

15. Procédé selon la revendication 14, l'agent thérapeutique étant la réolysine.


### SEQUENCE LISTING

<110> Expression Pathology Inc
<120> SRM/MRM Assay for the GTPase KRas Protein (KRas)
<130> 001152-8039.WO00
<150> 62/023,683
   <151> 2014-07-11
<160> 7
<170> PatentIn version 3.5
<210> 1
   <211> 9
   <212> PRT
   <213> Homo sapiens
<400> 1
   Ser Phe Glu Asp Ile His His Tyr Arg
   1               5
<210> 2
   <211> 12
   <212> PRT
   <213> Homo sapiens
<400> 2
   Gln Gly Val Asp Asp Ala Phe Tyr Thr Leu Val Arg
   1               5                   10
<210> 3
   <211> 11
   <212> PRT
   <213> Homo sapiens
<400> 3
   Leu Val Val Val Gly Ala Gly Gly Val Gly Lys
   1               5                   10
<210> 4
   <211> 25
   <212> PRT
   <213> Homo sapiens
<400> 4
   Ser Ala Leu Thr Ile Gln Leu Ile Gln Asn His Phe Val Asp Glu Tyr
   1               5                   10                  15
   Asp Pro Thr Ile Glu Asp Ser Tyr Arg
               20                  25
<210> 5
   <211> 7
   <212> PRT
   <213> Homo sapiens
<400> 5
   Gln Ala Gln Asp Leu Ala Arg
   1               5
<210> 6
   <211> 12
   <212> PRT
   <213> Homo sapiens
<400> 6
   Ser Tyr Gly Ile Pro Phe Ile Glu Thr Ser Ala Lys
   1               5                   10
<210> 7
   <211> 10
   <212> PRT
   <213> Homo sapiens
<400> 7
   Val Glu Asp Ala Phe Tyr Thr Leu Val Arg
   1               5                   10